# EUROPEAN PATENT APPLICATION

(11) **EP 1 826 570 A1**
(43) Date of publication of application: **29.08.2007**
(21) Application number: 05809362.6
(22) Date of filing: 18.11.2005
(51) Int. Cl.: G01N 33/72, G01T 1/161, G21G 4/08, C07K 14/805

(54) **REAGENT CONTAINING OXYGEN ISOTOPE-LABELED HEMOGLOBIN FOR EXAMINING VITAL TISSUE AND METHOD OF PRODUCING THE SAME**

(30) Priority: 22.11.2004 US 630257 P; 15.07.2005 US 699841 P
(71) Applicant: Oxygenix Co., Ltd., Tokyo 105-0001 (JP); University of Fukui, Fukui-shi, Fukui 910-8507 (JP)
(72) Inventor: Fujibayashi,Yasuhisa, Shimogyo-ku Kyoto-shi Kyoto 600-8212 (JP); Mori, Tetsuya, Fukui 910-0337 (JP); Suematsu, Makoto, Tokyo 167-0041 (JP); Ohta, Katsuji, Kawaguchi-shi Saitama 333-0862 (JP)
(74) Representative: Denison, Christopher Marcus
(86) International application number: PCT/JP2005/021637
(87) International publication number: WO 2006/054798

(57) **Abstract**

The present invention relates to a biological tissue examination agent comprising hemoglobin labeled with ¹⁵O₂ and a method for producing the same. More specifically, the present invention relates to a biological tissue examination agent for diagnosing a cause or condition of a disease by detecting oxygen metabolism in a biological tissue using hemoglobin labeled with ¹⁵O₂, and a method for producing the same. The present invention also relates to a method for detecting oxygen metabolism in a biological tissue.

## Description

### TECHNICAL FIELD

The present invention relates to a biological tissue examination agent comprising hemoglobin labeled with ¹⁵O₂ and a method for producing the same. More specifically, the present invention relates to a biological tissue examination agent for diagnosing a cause or condition of a disease by detecting oxygen metabolism in a biological tissue using hemoglobin labeled with ¹⁵O₂, and a method for producing the same. The present invention also relates to a method for detecting oxygen metabolism in a biological tissue.

### BACKGROUND ART

PET (Positron Emission Tomography) is a most advanced medical technology for diagnosing a cause or condition of a disease by labeling a bioactive substance and capturing a behavior thereof in a functional image.

According to a conventional technology for diagnosing, for example, brain using PET, labeled oxygen gas (¹⁵O₂) is inhaled through a nasal cavity (see, for example, "Clinical PET Handbook" 1st. edition published on October 30, 2001, edited by Kanji TORIZUKA, published by Institute for Techno-Economics). However, the following problems are pointed out for this method.
1) What amount of the inhaled ¹⁵O₂ is to be transferred to blood cannot be controlled, and also the using efficiency of ¹⁵O₂ is low.
2) The tracheae and lungs through which ¹⁵O₂ passes are exposed thereto, and thus the body is exposed to a large amount of ¹⁵O₂.
3) ¹⁵O₂ is used in a gaseous state and therefore has a high risk of leaking without being inhaled.

A method which is now receiving attention as being safer and more efficient than such a conventional method is administration by injection. According to this method, ¹⁵O₂ is dissolved in an erythrocyte solution and the resultant solution is administered by intravenous injection. This method, which allows direct administration of a high concentration of ¹⁵O₂ to blood, is expected to be superior to the conventional method on the following points.
1) Owing to the good transfer efficiency to the blood flow, the necessary amount of ¹⁵O₂ can be reduced.
2) The concentration in blood, and the administration dose, of ¹⁵O₂ can be more accurately adjusted.
3) The other organs are exposed to ¹⁵O₂ to a less extent.

For these reasons, technologies regarding erythrocyte containing dissolved ¹⁵O₂ have been actively developed recently as an administration method replacing inhalation. However, this method also has the following problems due to the use of an erythrocyte solution.
1) Autologous blood needs to be provided as an erythrocyte solution, which causes pain to the patient and increases the work amount for the medical staff.
2) When an erythrocyte preparation is used, there is a risk of virus infection or blood type incompatibility.
3) There is a risk that the medical staff may be infected with virus through the blood.
4) Human erythrocyte is individually indifferent. For example, the concentration of 2,3-bisphosphoglyceric acid or other intracellular substances involved in oxygen dissociation of hemoglobin is different among different individuals; and the oxygen release capability is lower in, for example, a diabetes patient due to the glycosylation reaction of globin. Therefore, erythrocyte is not desirable as an imaging carrier of PET which is required to have a high reproducibility.

In light of the above-described circumstances, a biological tissue examination agent which can release ¹⁵O₂ to a tissue or an organ by a safer and more efficient method and has a high reproducibility is desired to be developed.

Phillips WT, Lemen LD, Goins B et al., Amer J. Physiol 1997 describes that absorption of ¹⁵O₂-labeled hemoglobin into lungs and transfer thereof to a tissue were quantified. However, this article is regarding a measurement of the oxygen carrying ability, and they merely measured the carrying ability of hemoglobin encapsulated in liposome using ¹⁵O₂ in each of various tissues as an indicator.

### DISCLOSURE OF INVENTION

As a result of active studies and researches in light of such a situation, the present inventors found that use of an artificial oxygen carrier, instead of autologous blood or an erythrocyte preparation, allows the dynamics of ¹⁵O₂ in tissues and organs to be detected without causing the problems that the provision of autologous blood causes pain and the use of an erythrocyte preparation may cause virus infection. The present inventors thus completed the present invention.

The present invention is as follows.
(1) A biological tissue examination agent, comprising hemoglobin labeled with ¹⁵O₂.
(2) The biological tissue examination agent according to (1) above, wherein the hemoglobin labeled with ¹⁵O₂ is encapsulated in an artificial oxygen carrier.
(3) The biological tissue examination agent according to (1) or (2) above, wherein the hemoglobin labeled with ¹⁵O₂ is encapsulated in liposome.
(4) A biological tissue examination agent, comprising hemoglobin-encapsulating liposome encapsulating hemoglobin labeled with ¹⁵O₂.
(5) The biological tissue examination agent according to any one of (1) to (4) above, which is for positron emission tomography.
(6) A method for producing a biological tissue examination agent, comprising labeling hemoglobin to be encapsulated in hemoglobin-encapsulating liposome with ¹⁵O₂.
(7) The method according to (6) above, wherein the biological tissue examination agent is for positron emission tomography.
(8) A method for detecting oxygen metabolism in a biological tissue, which uses hemoglobin labeled with ¹⁵O₂.
(9) The method according to (8) above, wherein the hemoglobin labeled with ¹⁵O₂ is encapsulated in liposome.
(10) A use of hemoglobin labeled with ¹⁵O₂ for detecting oxygen metabolism in a biological tissue.
(11) The use according to (10) above, wherein the hemoglobin labeled with ¹⁵O₂ is encapsulated in liposome.

The present invention, which allows detection of oxygen metabolism in a tissue or an organ by a safe and efficient method, is widely usable for diagnosing a cause or condition of a disease of a patient.

### BRIEF DESCRIPTION OF DRAWINGS

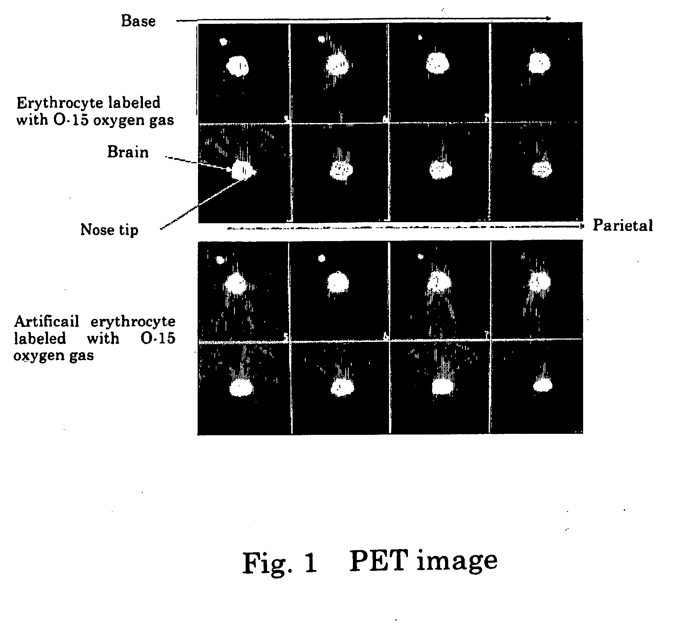

FIG. 1 shows PET examination results obtained by intravenous injection of ¹⁵O₂ gas-labeled erythrocyte and of an artificial oxygen carrier encapsulating ¹⁵O₂ gas-labeled hemoglobin into rabbits as test subjects.

### BEST MODE FOR CARRYING OUT THE INVENTION

Hereinafter, the present invention will be described in detail.

### 1. Biological tissue examination agent and method for producing the same

A biological tissue examination agent according to the present invention comprises hemoglobin labeled with ¹⁵O₂. The biological tissue examination agent of the present invention is for labeling a biological tissue or organ of a patient with ¹⁵O₂, and is used for detecting oxygen metabolism in the biological tissue or organ and thus diagnosing a cause or condition of a disease. The term "tissue" usually means a group of cells having the same function and form. In this specification, the term has a wider meaning and a "tissue" encompasses an organ comprising a combination of such groups of cells.

Hemoglobin used for the present invention is hemeprotein having a function of reversibly binding or dissociating oxygen, and is available from, for example, biologically derived erythrocyte, human erythrocyte derived from donated blood, or erythrocyte derived from domestic animals such as pigs, sheep, bovines and the like. The hemoglobin may be obtained by, for example, removing erythrocyte membrane (stroma) from erythrocyte by hypotonic hemolysis, heat-treating (at about 60°C for about 1 hour) the resultant substance to inactivate virus, and then purifying the resultant substance. For the present invention, gene-recombinant hemoglobin is usable after being purified and concentrated.

In the present invention, hemoglobin is used as being labeled with ¹⁵O₂. There is no specific limitation on the method for labeling hemoglobin. Hemoglobin may be labeled by, for example, bubbling ¹⁵O₂-containing gas in a liquid containing dispersed or floating hemoglobin. Hemoglobin may also be labeled using an artificial lung.

The time for bubbling depends on the content of ¹⁵O₂ in the gas, the apparatus used or the like. For example, when gas containing 5 to 20% of ¹⁵O₂ is used, the time for bubbling is usually about 0.1 to 2 minutes, and preferably about 60 seconds. A method for producing ¹⁵O₂ gas will be described later.

There is no specific limitation on the content of hemoglobin contained in the biological tissue examination agent of the present invention, and the amount of hemoglobin is usually in the range of 3 to 50 g/dL, preferably in the range of 5 to 25 g/dL, and more preferably in the range of 8 to 18 g/dL. There is no specific limitation on the amount of ¹⁵O₂ used for labeling hemoglobin, and the amount of ¹⁵O₂ is usually in the range of 3.7 GBq to 18.5 GBq, and preferably in the range of 5 GBq to 10 GBq per 1.0 g of hemoglobin.

In the present invention, hemoglobin labeled with ¹⁵O₂ is preferably encapsulated in an artificial oxygen carrier. This can suppress the side effects caused by the bioactivity of hemoglobin. The use of an artificial oxygen carrier also alleviates the burden on the patient or the work amount of the medical staff, and provides advantages of preventing the problems of virus infection and blood type incompatibility.

There is: no specific limitation on the artificial oxygen carrier usable for the present invention, and anything capable of reversibly absorbing or desorbing oxygen in a biological body is usable. Owing to such an arrangement, the tissue or organ can be labeled with ¹⁵O₂ gas. An artificial oxygen carrier usable for the present invention is, for example, a cell type oxygen infusion encapsulating hemoglobin in a phospholipid vesicle. As such a cell type oxygen infusion, hemoglobin-encapsulating liposome which encapsulates hemoglobin in an inner layer of a bimolecular structure thereof (hereinafter, such a hemoglobin-encapsulating liposome will occasionally be referred to as "HbV") is preferably used. In the present invention, a hemoglobin-encapsulating liposome in which at least a part of the hemoglobin is labeled with ¹⁵O₂ is preferably used. There is no specific limitation on the structure of the liposome used for the present invention. The liposome may be of a multi-layer type or a mono-layer type.

There is no specific limitation on the lipid of the phospholipid vesicle used for the present invention, and any known lipid is usable. Examples of usable lipids include glycolipid, sterols, fatty acid, phospholipid, amphilic alkylamino acid derivative, dialkyldimethylammonium, polyglycerolalkylether, polyoxyethylenealkylether and the like (Liposome Technology, 2nd edition, vol. 1, 141, 1993), alkylglycoside, alkylmethylglucamide, alkylsucrose ester and the like (Liposome, Technology, 2nd edition, vol. 1, 141, 1993), polyoxyethylene-polylactic acid and other amphilic block copolymers (Japanese PCT National-Phase Laid-Open Patent Publication No. 6-508831), long-chain alkylamines (tetradecylamine, hexadecylamine, stearylamine, etc.), and long-chain fatty acid hydrazides (hydrazide myristate, hydrazide palmitate, hydrazide stearate, etc.).

Examples of glycolipids usable for the present invention include glycoglycerolipid and glycosphingolipid. Examples of glycoglycerolipids include digalactosyldiglycerides (digalactosyldilauloylglyceride, digalactosyldimyristoylglyceride, digalactosyldipalmitoylglyceride, digalactosyldistearoylglyceride, etc.) and galactosyldiglycerides (galactosyldilauloylglyceride, galactosyldimyristoylglyceride, galactosyldipalmitoylglyceride, galactosyldistearoylglyceride, etc.). Examples of glycosphingolipids include galactosylcerebroside, lactosylcerebroside, ganglioside and the like.

Examples of sterols usable for the present invention include cholesterol, cholesterol hemisuccinate, 3β-[N-(N',N'-dimethylaminoethane)carbamoyl]cholesterol, ergosterol, lanosterol and the like.

Examples of phospholipids usable for the present invention include natural or synthetic phospholipids such as phosphatidylcholine, phosphatidylethanolamine, phosphatidylcerine, phosphatidylic acid, phosphatidylglycerol, phosphatidylinositol, lisophosphatidylcholine, sphingomyelin, egg yolk lecithin, soybean lecithin, hydrogennated phospholipid and the like.

In the present invention, one of these lipids, or a combination of a plurality of these lipids, may be used.

There is no specific limitation on the combining ratio of these lipids, and any known ratio is usable. For example, the ratio of phospholipid/cholesterol/fatty acid is, in terms of molar ratio, preferably 10/1 to 20/0.1 to 5, and more preferably 10/6 to 12/1.5 to 2.5.

The total amount of lipids contained in the phospholipid vesicle used for the biological tissue examination agent of the present invention is preferably about 0.1 to 2.0 (unit: g/g) based on the used amount of hemoglobin.

In the present invention, the surface of the phospholipid vesicle may be modified with polyalkyleneglycols. Since the residence time in blood of the biological tissue examination agent of the present invention can be extended by such an arrangement, the biological tissue examination agent can label the target tissue or organ more efficiently.

There is no specific limitation on the usable polyalkyleneglycols, and a polyalkyleneglycol including a alkylene chain having a carbon number of about 1 to 6 is preferable. The alkylene chain may be replaced with a substituent group providing no hindrance to the present invention, for example, a hydroxyl group, a carboxyl group, an amino group, alkoxy group or the like. Specifically, for example, polyethyleneglycol, polypropyleneglycol or the like is usable. There is no specific limitation on the molecular weight of the polyalkyleneglycols. A polyalkyleneglycol having a molecular weight of about 200 to 400 millions, preferably of about 1,000 to 50,000, is usable. In the present invention, there is no specific limitation on the content of the polyalkyleneglycols, and about 0.1 to 30 mol% based on the total amount of lipids included in the phospholipid vesicle is preferable.

The phospholipid vesicle usable for the present invention may be prepared by a known method, for example, reverse-phase evaporation, high-pressure extrusion, microfluidization or the like. Methods for modifying the surface of the phospholipid vesicle with a polyalkyleneglycol are also known. The surface of the phospholipid vesicle may usually be modified by, for example, using a bound lipid obtained by binding a non-phospholipid lipid and a polyalkyleneglycol via spacer or the like as a lipid forming the phospholipid vesicle.

According to one usable method for encapsulating hemoglobin labeled with ¹⁵O₂ in a phospholipid vesicle, when preparing the phospholipid vesicle, hemoglobin labeled with ¹⁵O₂ is added to a mixture solution of the lipid forming the phospholipid vesicle. However, considering that the half life of ¹⁵O₂ is short, the following method is more preferable. Hemoglobin-encapsulating liposome is prepared in advance using a non-labeled hemoglobin; and when used, ¹⁵O₂ gas is bubbled in the liquid containing dispersed or floating hemoglobin-encapsulating liposome, thus to label the hemoglobin with ¹⁵O₂.

The amount of the biological tissue examination agent of the present invention depends on the age, sex, body weight and symptom of the patient, site for examination, administration method (rapid or sustained) or the like, with no specific limitation. Usually, the radioactivity level of ¹⁵O₂ at the time of administration is preferably 18.5 MBq to 740 MBq, and is more preferably 37 MBq to 370 MBq.

A method for preparing hemoglobin-encapsulating liposome is more specifically described in Sakai H, et al.: "Hemoglobin-vesicles suspended in recombinant human serum albumin for resuscitation from hemorrhagic shock in anesthetized rats. Crit Care Med 2004 Vol. 32, No. 2, 539-545 (hereinafter, referred to as "Document 1 "). Document 1 is incorporated herein by reference.

The biological tissue examination agent of the present invention may optionally contain a known additive such as electrolyte, saccharide, amino acid, antioxidant, pH adjusting agent, isotonizing agent, or the like. Any additive which is usually used for medicine is usable with no specific limitation. One type of additive, or a combination of two or more types of additives, are usable. Such additives may be mixed with a lipid component forming the phospholipid vesicle at the time of preparation thereof and thus encapsulated in the phospholipid vesicle.

Examples of electrolytes usable for the present invention include sodium salts (e.g., sodium chloride, sodium hydrogen carbonate, sodium citrate, sodium lactate, sodium sulfate, sodium dihydrogenphosphate, disodium hydrogenphosphate, sodium acetate, sodium glycerophosphate, sodium carbonate, amino acid sodium salt, sodium propionate, sodium β-hydroxy butyrate, sodium gluconate), potassium salts (e.g., potassium chloride, potassium acetate, potassium gluconate, potassium hydrogen carbonate, potassium glycerophosphate, potassium sulfate, potassium lactate, potassium iodide, potassium dihydrogenphosphate, dipotassium hydrogenphosphate, potassium citrate, amino acid potassium salt, potassium propionate, potassium β-hydroxy butyrate), calcium salts (e.g., calcium chloride, calcium gluconate, calcium lactate, calcium glycerophosphate, calcium pantothenate, calcium acetate), magnesium salts (e.g., magnesium chloride, magnesium sulfate, magnesium glycerophosphate, magnesium acetate, magnesium lactate, amino acid magnesium salt), ammonium salts (e.g., ammonium chloride), zinc salts (e.g., zinc sulfate, zinc chloride, zinc gluconate, zinc lactate, zinc acetate), iron salts (e.g., iron sulfate, ferrous chloride, iron gluconate), copper salts (e.g., copper sulfate), manganese salts (e.g., manganese sulfate) and the like. Among these substances, sodium chloride, potassium chloride, magnesium chloride, disodium hydrogenphosphate, diopotassium hydrogenphosphate, potassium dihydrogenphosphate, sodium lactate, sodium acetate, sodium citrate, potassium acetate, potassium glycerophosphate, calcium gluconate, calcium chloride, magnesium sulfate, and zinc sulfate are especially preferable.

Examples of saccharides usable for the present invention include glucose, fluctose, xylitol, sorbitol, mannitol, dextrin, glycerin, sucrose, treharose, glycerol, maltose, lactose, erythritol and the like. Among these substances, glucose, fluctose, xylitol, sorbitol, mannitol, dextrin, glycerin, and sucrose are especially preferable.

Examples of amino acids usable for the present invention include lysine, lysine chloride, lysine acetate, asparagine, glutamine, asparaginic acid, glutaminic acid, serine, threonine, tyrosine, methionine, cystine, cysteine, cysteine chloride, cysteine malate, homocysteine, isoleucine, leucine, phenylalanine, tryptophan, varin, arginine, arginine chloride, histidine, histidine chloride, alanine, proline, aminoacetic acid and the like, and salts thereof. Among these substances, lysine, asparagine, glutamine, asparaginic acid, glutaminic acid, serine, threonine, tyrosine, methionine, cystine, cysteine, homocysteine, and aminoacetic acid are especially preferable.

Examples of antioxidants usable for the present invention include sodium hydrogen sulfite, sodium sulfite, sodium pyrosulfite (e.g., sodium metabisulfite), Rongalit (CH₂OHSO₂Na), ascorbic acid, sodium ascorbate, erythorbic acid, sodium erythorbate, cysteine, cysteine chloride, homocysteine, glutathione, thioglycerol, alfathioglycerin, sodium edetate, citric acid, isopropyl citrate, potassium dichloroisocyanurate, sodium thioglycolate, sodium thiomalate, sodium pyrosulfite, 1,3-butyleneglycol, calcium disodium ethylenediaminetetraacetate, disodium ethylenediaminetetraacetate, amino acid sulfite (e.g., L-lysine sulfurous acid), butylhydroxyanisol (BHA), dibutylhydroxytoluene (BHT), propyl gallate, palmitate ascorbate, vitamin E and derivatives thereof (e.g., d1-α-tocopherol, tocopherol acetate, natural vitamin E, d-δ-tocopherol, concentrated mixed tocopherol, trolox), guaiac gum, nordihydro-guaiaretic acid (NDGA), L-ascorbic acid stearic acid ester, soybean lecitin, palmitic acid ascorbic acid, benzotriazol, pentaerythrityl-tetrakis [3-(3,5-di-t-butyl-4-hydroxyphenyl)propionate]2-mercaptobenzimidazole and the like. Among these substances, sodium hydrogen sulfite, sodium sulfite, ascorbic acid, homocysteine, d1-α-tocopherol, tocopherol acetate, glutathione, and trolox are especially preferable.

Examples of pH adjusting agents usable for the present invention include the following. Examples of acids include adipic acid, casein sodium, hydrochloric acid, diluted hydrochloric acid, sulfuric acid, aluminum potassium sulfate, citric acid, sodium dihydrogen citrate, glycine, glucono-δ-lactone, gluconic acid, sodium gloconate, crystalline sodium dihydrogenphosphate, succinic acid, acetic acid, ammonium acetate, tartaric acid, D-tartaric acid, lactic acid, glacial acetic acid, monosodium fumarate, sodium propionate, boric acid, ammonium borate, maleic acid, malonic acid, malic acid, anhydrous disodium phosphate, methanesulfonic acid, phosphoric acid, dihydrogenphosphoate (e.g., potassium dihydrogenphosphate, sodium dihydrogenphosphate) and the like. Among these substances, hydrochloric acid, succinic acid, acetic acid, lactic acid, glacial acetic acid, phosphoric acid, potassium dihydrogenphosphate, and sodium dihydrogenphosphate are preferable.

Examples of alkalis include ammonia water, dry sodium carbonate, sodium citrate, sodium acetate, diisopropanolamine, L-sodium tartrate, lactate (e.g., calcium lactate, sodium lactate), borax, sodium maleate, sodium malonate, sodium malate, potassium hydroxide, calcium hydroxide, sodium hydroxide, magnesium hydroxide, sodium hydrogen carbonate, sodium carbonate, triisopropanolamine, monoethanolamine, triethanolamine, anhydrous sodium acetate, anhydrous sodium monohydrogenphosphate, meglumine, phosphate (e.g., trisodium phosphate), sodium salt of barbital, hydrogen phosphate (e.g., disodium hydrogenphosphate, dipotassium hydrogenphosphate) and the like. Among these substances, sodium acetate, sodium hydroxide, sodium hydrogen carbonate, sodium carbonate, trisodium phosphate, disodium hydrogenphosphate, and dipotassium hydrogenphosphate are especially preferable.

Examples of isotonizing agents usable for the present invention include aminoethylsulfonic acid, sodium hydrogen sulfite, potassium chloride, calcium chloride, sodium chloride, benzalkonium chloride, magnesium chloride, saccharides (e.g., lactose, concentrated glycerin, dextrose, fructose, xylitol, glycerin), sugar alcohol (e.g., D-sorbitol, D-mannitol), citric acid, sodium citrate, crystalline sodium dihydrogenphosphate, calcium bromide, sodium bromide, sodium hydroxide, physiological saline, sodium tartrate-dihydrate, sodium hydrogen carbonate, nicotinamide, sodium lactate solution, propyleneglycol, benzylalcohol, boric acid, borax, anhydrous sodium pyrophosphate, phosphoric acid, disodium hydrogenphosphate, potassium dihydrogenphosphate, sodium dihydrogenphosphate, and macrogol 4000. Among these substances, potassium chloride, sodium chloride, concentrated glycerin, disodium hydrogenphosphate, and potassiumdihydrogen phosphate are especially preferable.

The biological tissue examination agent of the present invention is widely usable for an examination method using detection of oxygen metabolism in a biological tissue or organ. For example, the biological tissue examination agent of the present invention is preferably usable for PET examination. By using the biological tissue examination agent of the present invention, the problems involved in the conventional method of causing ¹⁵O₂ gas to be inhaled through a nasal cavity of the patient are solved. Thus, the examination can be performed safely and efficiently. According to the PET examination, the behavior of a tissue or organ of the patient can be taken in a tomograph, and a cause or condition of the disease can be accurately diagnosed. For these reasons, the biological tissue examination agent of the present invention usable for the PET examination is highly useful. Examples of diseases which can be diagnosed by the biological tissue examination agent of the present invention include cancer, isochaemic circulatory disturbance (cerebral infarction, myocardial infarction, unstable cardiomyopathy, etc.), subarachnoid hemorrhage, moyamoya disease, cerebral hemorrhage and the like. The biological tissue examination agent of the present invention is usable for diagnosing these diseases.

Next, a method for producing the biological tissue examination agent of the present invention will be described. A method according to the present invention comprises labeling hemoglobin to be encapsulated in hemoglobin-encapsulating liposome with ¹⁵O₂. The method for labeling hemoglobin with ¹⁵O₂ is as described above. Considering that the half life of ¹⁵O₂ is short, it is preferable that the hemoglobin is labeled with ¹⁵O₂ by, after a hemoglobin-encapsulating liposome is prepared, bubbling ¹⁵O₂ gas in the liquid containing dispersed or floating the hemoglobin-encapsulating liposome. The hemoglobin-encapsulating liposome may be produced by any known method as described above.

The production of the biological tissue examination agent of the present invention is preferably performed in a sterile state. The biological tissue examination agent produced by a method of the present invention is widely usable for an examination using detection of oxygen metabolism, and is especially preferably usable for PET examination.

### 2. A method for detecting oxygen metabolism in a biological tissue

Next, a method for detecting oxygen metabolism in a biological tissue according to the present invention will be described. A method for detecting oxygen metabolism of the present invention comprises using hemoglobin labeled with ¹⁵O₂. According to a preferable embodiment of the present invention, hemoglobin in an artificial oxygen carrier is labeled using ¹⁵O₂, and the dynamics of ¹⁵O₂ in a tissue or organ of a test subject can be detected using the labeled hemoglobin. More preferably, the labeled hemoglobin is administered into the body of the test subject by intravenous injection.

In the detection method of the present invention, the hemoglobin labeled with ¹⁵O₂ is introduced into a tissue or organ while being encapsulated preferably in an artificial oxygen carrier, more preferably in liposome. As the hemoglobin labeled with ¹⁵O₂ usable for the present invention, any biological tissue examination agent of the present invention described above is usable. For example, by using a hemoglobin-encapsulating liposome encapsulating hemoglobin labeled with ¹⁵O₂, the side effects caused by hemoglobin can be suppressed. The amount of hemoglobin labeled with ¹⁵O₂ in the detection method of the present invention is defined by the content of hemoglobin and the amount of ¹⁵O₂ used for labeling hemoglobin, as described above regarding the biological tissue examination agent.

According to a preferable embodiment of the present invention, a method of the present invention comprises the steps of (a) producing ¹⁵O₂ gas, (b) labeling hemoglobin in an artificial oxygen carrier with ¹⁵O₂ gas, (c) administering the hemoglobin labeled with ¹⁵O₂ gas to the body, and (d) detecting ¹⁵O₂ gas. Hereinafter, each step will be described.
(a) Step of producing ¹⁵O₂ gas
   In this step, ¹⁵O₂ gas is produced. ¹⁵O₂ gas may be produced by, for example, radiating deuteron to nitrogen 14 in a cyclotron, through ¹⁴N(d, n)¹⁵O reaction. Nitrogen 14 may be obtained by a N₂ generator, or produced through ¹⁵N(p, n)¹⁵O reaction. ¹⁵N may be obtained by isotope concentration.
   In the present invention, ¹⁵O₂ produced by the method described above may optionally be purified. ¹⁵O₂ gas is preferably purified using a molecular sieve. By such purification, the concentration of ¹⁵O₂ is increased and more radioactivity is detected. It should be noted that since the half life of ¹⁵O₂ is short, the radioactivity level may possibly be lowered by purification. Therefore, it is desirable to determine whether or not to purity ¹⁵O₂ in consideration of the balance with the concentration efficiency or purification time.
(b) Step of labeling hemoglobin in an artificial oxygen carrier with ¹⁵O₂ gas
   In this step; the hemoglobin in the artificial oxygen carrier is labeled with ¹⁵O₂ gas. The hemoglobin in the artificial oxygen carrier may be labeled with ¹⁵O₂ gas by, for example, bubbling ¹⁵O₂ gas (containing 5 to 20% of ¹⁵O₂ gas) in a liquid containing dispersed or floating HbV (deoxyHb type) for about 0.1 to 2 minutes, preferably for about 60 seconds.
   There is no specific limitation on the amount of ¹⁵O₂, and the amount is usually in the range of 3.7 GBq to 18.5 GBq per 1 g of hemoglobin. As can be understood, according to the method of the present invention, the using efficiency of ¹⁵O₂ is higher than the inhalation method.
   There is no specific limitation on the artificial oxygen carrier usable for the present invention, and anything capable of reversibly absorbing or desorbing oxygen in a biological body is usable. A cell type oxygen infusion encapsulating hemoglobin in a phospholipid vesicle is preferably used. More preferably, HbV is used.
   An artificial oxygen carrier has the following advantages over an erythrocyte preparation.
   1) Can inactivate virus and can be purified, and therefore is highly safe.
   2) Can be stored for a long time (desirably for 2 years) at room temperature, and therefore can be prepared when necessary in a necessary amount.
   3) Has no risk of blood type incompatibility.
   4) Does not perform any interaction with other blood components.
   5) Does not contain protein in a solution and therefore does not need a special device such as an artificial lung or the like.
(c) Step of administering the hemoglobin labeled with ¹⁵O₂ gas to the body
   In this step, the hemoglobin labeled with ¹⁵O₂ gas is administered to the body. The administration may be conducted by sampling the artificial oxygen carrier encapsulating the hemoglobin labeled with ¹⁵O₂ in step (b) above using, for example, an injection needle and a syringe and injecting the artificial oxygen carrier by intravenous injection.
(d) Step of detecting ¹⁵O₂ gas
   In this step, ¹⁵O₂ is detected. The detection is preferably conducted by, for example, using a PET apparatus. By this, the oxygen metabolism condition in each tissue can be detected and thus the ischemia condition or the like can be diagnosed. Any known PET apparatus is usable.
   In the usual PET examination, the radioactivity level generally used is about 200 to 400 MBq. Considering this, it is preferable that the radioactivity level used in the present invention at the time of labeling is usually in the range of 200 MBq to 2000 MBq.
   The radiochemical half life of ¹⁵O is 2.04 min (β+, electron capture). Considering this and also the labeling efficiency, the radioactivity level changes, for example, as follows in the steps (a) through (d) above.
   (a) 40 GBq → (b) 10 GBq → (c) 500 MBq → (d) 200 MBq

According to a preferable embodiment of the present invention, the method of the present invention comprises steps (a) through (d) above, so that the oxygen metabolism condition in each tissue (for example, brain) can be detected and the ischemia condition or the like can be diagnosed.

According to a preferable embodiment of the present invention, an artificial oxygen carrier is used, instead of autologous blood or an erythrocyte preparation, as a bioactive substance to be labeled. Therefore, the dynamics of ¹⁵O₂ can be detected in a tissue or organ of a test subject by a highly safe and simple method without imposing any burden on the patient or the medical staff. The artificial oxygen carrier, which does not have a risk of blood type incompatibility and does not perform any interaction with other blood components, has an advantage of allowing diagnose by a simple method without considering these factors. The artificial oxygen carrier also has big advantages of being stored in a long time at room temperature and of realizing oxygen metabolism imaging without relying on erythrocyte, which is functionally quite different among individuals.

This application is filed claiming the benefit of priority based upon U.S. provisional application S.N. 60/630,257 filed on November 22, 2004 and U.S. provisional application S.N. 60/699,841 filed on July 15, 2005, both of which are incorporated herein by reference.

### EXAMPLES

The present invention will be further described in more detail in the following Examples, which are not intended to limit the scope of the invention.
1. 6 ml of blood was put into a 15 ml vial, and radioactive gas (containing about 20% of O₂) was bubbled for about 20 seconds through an injection needle inserted to the bottom thereof. The exhaust gas was recovered to a drain via another needle separately provided.
2. In the state where the vial contains the radioactive level of 185 MBq, all the blood was sampled using an injection needle and a syringe connected by a line.
3. The radioactive level in the syringe was measured (18.5 MBq), and the blood was immediately injected through phleboclysis via a line installed on rabbit ears. PET data was collected for 10 minutes from the start of the injection. The results are shown in the upper half of FIG. 1. As the PET apparatus, Advance produced by General Electric Company was used.
4. Substantially the same experiment was performed using an artificial oxygen carrier, and data was collected. The obtained images are shown in the lower half of FIG. 1. As the artificial oxygen carrier, a sample prepared by the method described in Document 1 was used. The specific method for preparation is described below.
5. The PET measurement using the artificial oxygen carrier provided exactly the same images as those obtained using blood (erythrocyte). Thus, it was confirmed that an artificial oxygen carrier is effectively usable for the PET examination.

### <Preparation of an artificial oxygen carrier>

A mixture containing 1,2-dipalmitoyl-sn-glycero-3-phosphatidylcholine, cholesterol, and 1,5-bis-O-hexadecyl-N-succinyl-L-glutamate (produced by Nippon Fine Chemical Co., Ltd.) at a molar ratio of 5/5/1 and containing 1,2-distearoyl-sn-glycero-3-phosphatidylethanolamine-N-poly(ethyleneglycol) (produced by NOF Corporation) at 0.3 mol% based on the total lipid amount was put into a round bottomed flask. Benzene was added thereto, and the resultant substance was completely dissolved while being heated. The resultant substance was frozen by liquid nitrogen, put to a lyophilization apparatus, and lyophilized for 12 hours. As a result, white powder was obtained.

The white powder was added to injection water and stirred at 25°C to obtain a dispersant containing vesicle having a particle diameter of 1.8 µm. A freezing and melting cycle by which the dispersant was frozen by liquid nitrogen and melted at 25°C was repeated 4 times. Thus, a dispersant containing vesicle having a particle diameter of 520 nm was obtained. The dispersant was frozen by liquid nitrogen, and put to a lyophilization apparatus, and lyophilized for 15 hours. As a result, dry white vesicle was obtained.

Hemoglobin (40 g/dL) to be encapsulated in an artificial oxygen carrier was obtained by purifying erythrocyte derived from donated blood. For adjusting the allosteric effect, pyridoxal 5'-phosphate (produced by Sigma Chemical) was added at 2.5 molar ratio with respect to the hemoglobin.

5 mL of a hemoglobin solution was added to the dry vesicle and stirred at 25°C to obtain a dispersant containing hemoglobin vesicle. At this point, the particle diameter of the vesicle was 540 nm, which was substantially the same as the pre-drying particle diameter. The dispersant was put to EXTRUDER (registered trademark) (trade name; produced by Nichiyu Liposome K.K.) and sequentially passed through acetylcellulose filters having pore diameters of 3.0 µm, 0.8 µm, 0.65 µm, 0.45 µm, 0.30 µm and 0.22 µm (produced by Fujifilm Corporation) at 14°C while being pressurized (20 kg/cm²). Thus, a dispersant containing hemoglobin vesicle was obtained. The dispersant was passed through an ultrafiltration membrane to remove unencapsulated hemoglobin.

The dispersant containing hemoglobin vesicle was put into a cylindrical flask. The flask was put to a rotary evaporator and rotated (56 rpm). The liquid membrane formed by this was irradiated with visible light using a halogen lamp (500 W) for 3 minutes in ventilating oxygen (1 L/min.). Thus, ligand exchange was performed to convert carbon monoxide-bound hemoglobin (HbCO) into oxyhemoglobin (HbO₂). The oxyhemoglobin was sealed in a vial, and then vapor-saturated nitrogen gas was introduced into the vial to bubble the nitrogen gas in the hemoglobin vesicle liquid. Thus, the dissolved oxygen was removed, and as a result, the artificial oxygen carrier was obtained.

### INDUSTRIAL APPLICABILITY

The present invention uses the detection of oxygen metabolism in a tissue or organ of a patient and therefore is widely usable for an examination method for diagnosing a cause or condition of a disease of the patient.

The present invention, when used in combination with ¹¹C-flurodeoxyglucose generally used for detecting cancer lesions, allows qualitative diagnose of cancer lesions which actively absorb oxygen, and therefore is applicable to find the presence/absence of radioactive sensitivity.

The present invention is useful for diagnosing an area which is around the site of neurocyte death after cerebral infarction but can be cured by treatment.

## Claims

1. A biological tissue examination agent, comprising hemoglobin labeled with ¹⁵O₂.

2. The biological tissue examination agent according to claim 1 or 2, wherein the hemoglobin labeled with ¹⁵O₂ is encapsulated in an artificial oxygen carrier.

3. The biological tissue examination agent according to claim 1, wherein the hemoglobin labeled with ¹⁵O₂ is encapsulated in liposome.

4. A biological tissue examination agent, comprising hemoglobin-encapsulating liposome encapsulating hemoglobin labeled with ¹⁵O₂.

5. The biological tissue examination agent according to any one of claims 1 to 4, which is for positron emission tomography.

6. A method for producing a biological tissue examination agent, comprising labeling hemoglobin to be encapsulated in hemoglobin-encapsulating liposome with ¹⁵O₂.

7. The method according to claim 6, wherein the biological tissue examination agent is for positron emission tomography.

8. A method for detecting oxygen metabolism in a biological tissue, which uses hemoglobin labeled with ¹⁵O₂.

9. The method according to claim 8, wherein the hemoglobin labeled with ¹⁵O₂ is encapsulated in liposome.

10. A use of hemoglobin labeled with ¹⁵O₂ for detecting oxygen metabolism in a biological tissue.

11. The use according to claim 10, wherein the hemoglobin labeled with ¹⁵O₂ is encapsulated in liposome.
